# EUROPEAN PATENT APPLICATION

(11) **EP 2 743 678 A1**
(43) Date of publication of application: **18.06.2014**
(21) Application number: 12821746.0
(22) Date of filing: 07.08.2012
(51) Int. Cl.: G01N 21/35

(54) **INFRARED ANALYSIS OF PERIPHERAL BLOOD FRACTIONS OBTAINED TO INDICATE COGNITIVE DEVELOPMENT ASSOCIATED WITH ALZHEIMER'S DISEASE**

(30) Priority: 08.08.2011 ES 201131370 P
(71) Applicant: Consejo Superior De Investigaciones Científicas (CSIC), 28006 Madrid (ES); Instituto de Salud Carlos III, 28019 Madrid (ES); Fundación Cien, 28031 Madrid (ES); Fundación Para La Investigación Biomédica Del Hospital 12 de Octubre, 28041 Madrid (ES)
(72) Inventor: CARMONA HERNÁNDEZ, Pedro, E-28006 Madrid (ES); TOLEDANO GASCA, Adolfo, E-28002 Madrid (ES); CALERO LARA, Miguel, E-28019 Madrid (ES); MARTÍNEZ MARTÍN, Pablo, E-28019 Madrid (ES); BERMEJO PAREJA, Félix, E-Madrid E-28041 (ES)
(74) Representative: ABG Patentes, S.L.
(86) International application number: PCT/ES2012/070613
(87) International publication number: WO 2013/021087

(57) **Abstract**

The present invention relates to a method for transmission infrared spectroscopic analysis for determining the degree of global cognitive impairment associated with Alzheimer's disease based on analyzing the 3100-1300 cm⁻¹ infrared spectrum region of a previously obtained and fractionated sample of blood. Preferably, the fractionated sample of blood to be analyzed is a fraction of leukocytes.

Likewise, another object of the present invention relates to the use of said method to obtain an indication of the status of the Alzheimer's disease and/or an indication of the degree of cognitive impairment associated with said disease.

## Description

### Technical Field

The invention relates to a new method of determining the β-sheet protein structure in the leukocytes of collected peripheral blood by means of transmission infrared spectroscopy, and the main field of application is the health field, and it may also be of interest to the commercial field of infrared spectroscopy instruments. More specifically, the invention relates to a method for indicating the presence or absence of Alzheimer's disease (AD) and/or the degree of progression thereof, based on transmission infrared spectroscopy.

### State of the Art

In the next 50 years, Alzheimer's disease (AD) is expected to affect 100 million people worldwide. It is well known that the most important pathophysiological characteristics of Alzheimer's disease are the senile plaques and the neurofibrillary tangles. The majority component of said plaques is the amyloid-β peptide (Aβ). This peptide containing 39-43 amino acids is highly hydrophobic, is released in the proteolytic cleavage of the amyloid precursor protein (APP), and aggregates to form oligomers. These oligomers in turn aggregate to form fibers, which are then deposited in the brain parenchyma and cause senile plaques (Haass EMBO Journal 23, 483-488 (2004)). The neurotoxic effects of the Aβ peptide also involve changes in the composition and structure of the neuronal membranes, affecting their physicochemical properties. One of the biggest challenges of this neurodegenerative disease lies in achieving a method of early diagnosis in order to administer suitable treatment. The most effective definitive diagnosis today is limited to a *post-mortem* examination, and clinical diagnosis is done by exclusion after the specialist studies whether dementia exists and finds no symptom or sign explaining the cause for said dementia. Immunochemical methods using antibodies are known to be tedious, and furthermore they do not have 100% sensitivity due to the possible existence of several structural isoforms of the biomarkers. Many problems are raised with this disease, including among such problems the early diagnosis and follow-up of the patients' progress, as well as the definitive post-mortem diagnosis and the characterization of neuropathological changes. All these problems require developing low-cost, sensitive and reliable methods that are easy to perform and repeat.

In this regard, infrared spectroscopy has been a well-established technique in industrial and scientific research areas for several decades. Its advantages over other common techniques are:
a. Samples in any physical condition can be analyzed with a simple and relatively rapid preparation, unlike other analytical techniques.
b. Relatively small amounts of sample are required.

This spectroscopic technique has recently been applied to cerebrospinal fluid analysis for diagnosing Alzheimer's disease (Griebe et al. Neuroscience Letters 420, 29-33 (2007)), with a sensitivity and specificity of about 85 and 75%, respectively. However, it is not as easy to draw this biological fluid as it is to draw blood, not to mention the psychological connotations of drawing cerebrospinal fluid. Infrared spectroscopy has also been applied to the analysis of brain tissue collected from patients with Alzheimer's disease (Rak et al. Biopolymers 78, 207-217 (2007); Choo et al. Biophysical Journal 71, 1672-1679 (1996); Fabian et al., Applied Spectroscopy 47, 1513-1518 (1993)), obtaining spectra with bands characteristic of amyloid peptides located in the spectral range of 1635-1624 cm⁻¹. Therefore, there is no research in the literature where infrared spectroscopy is applied to the analysis of blood components or fractions as a criterion for diagnosing Alzheimer's disease. Given the possibility that this technique has of determining the composition and structure of cellular and acellular components of the blood, which is a biological fluid that is easy to draw, and of having the aforementioned advantages over other techniques, it has been applied in this invention as method of diagnosing this disease.

### Description of the Invention

### Brief Description of the Invention

The present invention relates to a method of applying transmission infrared spectroscopy to the analysis of samples of collected blood, preferably collected human peripheral blood, for determining the global cognitive impairment associated with Alzheimer's disease, and therefore, for applying it to the diagnosis of Alzheimer's disease. The method requires prior fractionation of the samples of collected blood, which are collected in BD Vacutainer CPT tubes with sodium citrate and subjected to centrifugation-filtration.

Several microliters of the aqueous suspension containing leukocytes are deposited on a glass support, for example a zinc selenide glass support, for samples intended for infrared spectroscopy, for the purpose of obtaining a film made of these cells and measuring its corresponding spectrum. Other water-insoluble sample supports such as calcium fluoride, zinc sulfide, barium fluoride supports, can also be used. The measured spectrum is mathematically processed for determining the corresponding concentration of β-sheet protein structure, which is associated with AD, and furthermore subjecting it to multivariate statistical analysis (PLS). For sensitivity reasons, said protein structure has been determined by means of the infrared spectra expressed in second derivatives. PLS analysis is based on correlating the parameter or scale defining the different stages of Alzheimer's disease (Global Deterioration Scale, GDS) with spectral regions that are connected with this disease, namely, the amide I region (1700-1600 cm⁻¹), the amide II region (1600-1500 cm⁻¹), the region comprised between 1500-1300 cm⁻¹ (including bands of C-H angle deformation and stretching of carboxylate groups) and the C-H stretching region comprised between 3100 and 2800 cm⁻¹. Finally, the percentage of prediction in samples from healthy controls and from patients is measured to obtain the sensitivity and specificity of the method. A more detailed description of the invention is provided below, where several drawings and illustrative examples are described.

### Detailed Description of the Invention

The present invention is based on the fact that the inventors have observed, on one hand, that the β-sheet protein structure is more abundant in leukocytes collected from patients with AD than in leukocytes collected from controls, and, on the other hand, that there is a certain correlation between the Global Deterioration Scale (GDS) of this disease and the spectral profiles of the amide I region (1700-1600 cm⁻⁻¹), amide II region (1600-1500 cm⁻¹), the region comprised between 1500-1300 cm⁻¹ (including bands of C-H angle deformation and stretching of carboxylate groups) and the C-H stretching region comprised between 3100 and 2800 cm⁻¹.

In the field of the present invention, the Global Deterioration Scale (also herein referred to as GDS) refers to Reisberg's Global Deterioration Scale for describing the stages of progression of Alzheimer's disease in detail. Said scale consists of seven levels, and in the field of the present invention are defined as:
- GDS-1: corresponding to a normal individual with no cognitive deficit and a normal clinical phase;
- GDS-2: corresponding to an individual with a very mild level of cognitive deficit (subjective cognitive impairment) and a clinical phase of forgetfulness;
- GDS-3: corresponding to an individual with a mild level of cognitive deficit and a clinical phase of early confusion;
- GDS-4: corresponding to an individual with a moderate level of cognitive deficit and a clinical phase of late confusion;
- GDS-5: corresponding to an individual with a moderately severe level of cognitive deficit and a clinical phase of initial dementia;
- GDS-6: corresponding to an individual with a severe level of cognitive deficit and a clinical phase of mild dementia;
- GDS-7: corresponding to an individual with a very severe level of cognitive deficit and a clinical phase of severe dementia.

For the purposes of this specification, GDS-3 is considered a mild level of progression of Alzheimer's disease (mild AD), GDS-4 and GDS-5 are considered a moderate level of progression of Alzheimer's disease (moderate AD) and GDS-6 and GDS-7 are considered an advanced, serious or severe level of progression of Alzheimer's disease (severe AD).

Therefore, a first aspect of the present invention relates to an infrared spectroscopic method for determining the global cognitive impairment associated with Alzheimer's disease comprising the following steps:
a. recording a transmission infrared spectrum of a first sample of collected blood;
b. obtaining at least a first numerical parameter from at least one signal of the spectrum of the region comprised between 1300 and 3100 cm⁻¹, including both limits;
c. comparing said at least first numerical parameter of step b with at least a second reference numerical parameter;
d. determining based on the comparison in the preceding step if the sample of blood of step a corresponds to a class defined by:
   I. no cognitive impairment,
   II. cognitive impairment associated with Alzheimer's disease.

In one embodiment, the sample of blood that is analyzed is a sample of human peripheral blood. Preferably, said sample is a fraction of blood derived from the fractionation of said collected blood, and more preferably, it is a cellular fraction (such as for example erythrocytes, leukocytes or thrombocytes) or an acellular fraction (blood plasma) from human peripheral blood. In an even more preferred embodiment, it is a fraction of leukocytes from human peripheral blood.

Generally and in a non-limiting manner, the sample of the fraction of collected blood, suitable for the infrared spectroscopic analysis of the present invention, can be prepared before acquiring the spectroscopic data according to a method comprising the following steps:
a. preparing an aqueous suspension of a fraction of collected blood by centrifugation-filtration,
b. depositing and spreading a volume of the aforementioned aqueous suspension comprised between 5 and 125 µL, including both limits, and more preferably between 75 and 125 µL, including both limits, over a zinc selenide glass;
c. completely evaporating the deposited suspension at a low temperature, preferably at temperatures comprised between 2 and 20°C, including both limits, and more preferably between 2 and 10°C, including both limits.

In a different preferred embodiment, the first parameter of the spectral region comprised between 3100 and 1300 cm⁻¹ is a percentage of β-peptide structure content of the sample of collected blood to be analyzed.

To calculate the percentage of β-peptide structure content, the general method can comprise the following steps:
(a) expressing the infrared spectrum 1700-1600 cm⁻¹ region (including both limits) of the test sample of collected blood in the second derivative in the frequency domain;
(b) identifying each of the bands associated with β-peptide type structure in the infrared spectrum in the region comprised between 1639-1623 cm⁻¹ (including both limits), the frequency values of which coincide with those where local minima appear in said spectrum expressed in the second derivative; and
(c) multiplying by 100 the sum of the areas of each of the bands associated with a β-peptide type structure in the infrared spectrum region comprised between 1639 and 1623 cm⁻¹ (including both limits), and dividing by the total area of said spectrum in the region comprised between 1680 and 1623 cm⁻¹ (including both limits).

Alternatively and for the same purpose, the method can also proceed like in steps (a), (b) and (c) described above, but considering in step (a) the infrared spectrum resulting from a Fourier deconvolution with the factor y = 10.

Said percentage of β-peptide structure content of the test sample of collected blood can be used as a cut-off point for classifying said sample in class I or in class II, as defined above, by comparison with a reference numerical parameter, used as the limit value between both classes. This limit reference numerical parameter is the result of experimental statistical studies (as described in Example 3). Therefore, if the percentage of β-peptide structure content is equal to or less than 12%, the test sample of collected blood belongs to class I (without cognitive impairment), and if it is greater than 12%, said sample of blood belongs to class II (cognitive impairment associated with Alzheimer's disease). According to this classification of the first infrared spectrum, a result is obtained with about 80% sensitivity and 75% specificity. This sensitivity refers to the probability that a class II sample subjected to analysis by this method will give a positive result for class II, and in percentage terms it is calculated by multiplying by 100 the quotient resulting from dividing the number of hits of class II samples by the total number of class II samples analyzed. In terms of specificity, it refers to the probability that a class I sample subjected to said analysis will give a positive result for class I, and in percentage terms it is calculated by multiplying by 100 the quotient resulting from dividing the number of hits of class I samples by the total number of class I samples analyzed.

In another preferred embodiment other than those described above, said at least second reference numerical parameter is obtained under the same conditions as the first numerical parameter of the first spectrum, corresponding to the test sample of blood, from a second recorded infrared spectrum of at least a second reference sample of blood collected from each of the classes defined in I and II.

In a more preferred embodiment than the preceding one:
- class I of said second reference sample of blood comprises at least the subclass defined by I-a, corresponding to a subclass of no cognitive impairment, without Alzheimer's disease, with a value of GDS-1 on Reisberg's global deterioration scale,
- and class II of said second reference sample of blood comprises at least the subclasses defined by:
   II-a. mild cognitive impairment associated with Alzheimer's disease with a value of GDS-3 on Reisberg's global deterioration scale,
   II-b. moderate cognitive impairment associated with Alzheimer's disease with a value of GDS-4 on Reisberg's global deterioration scale,
   II-c. moderately severe cognitive impairment associated with Alzheimer's disease with a value of GDS-5 on Reisberg's global deterioration scale,
   II-d. severe cognitive impairment associated with Alzheimer's disease with a value of GDS-6 on Reisberg's global deterioration scale,
   II-e. very severe cognitive impairment associated with Alzheimer's disease with a value of GDS-7 on Reisberg's global deterioration scale.

More preferably, said first numerical parameter is the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of the first sample of blood, and said second reference numerical parameter of each of the subclasses defined in I-a, II-a, II-b, II-c, II-d and II-e is the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of each reference sample of blood. In an even more preferred embodiment, said absorbance values are determined from the spectrum previously normalized with respect to the absorbance of the amide I band in the 1700-1600 cm⁻¹ region, and referring to one and the same absorbance value in the 1800 cm⁻¹ frequency.

Even more preferably, the comparison step comprises a multivariate analysis of partial least squares (PLS) using:
- the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of the first sample of collected blood and the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of each reference sample of collected blood as independent variables;
- and the numerical GDS value on Reisberg's global deterioration scale corresponding to the subclass as defined in I-a, II-a, II-b, II-c, II-d and II-e of said second reference sample of blood as a dependent variable.

Without limiting the scope of the invention and by way of example, the method of the invention can comprise the following steps:
1) Collecting a fraction of leukocytes from peripheral blood by means of centrifugation-filtration.
2) Obtaining a leukocyte film on a zinc selenide glass suitable for measuring transmission infrared spectra.
3) Measuring infrared spectra of the leukocyte film and then mathematically processing the spectra to: (a) determine the β-sheet protein structures; and (b) perform the PLS analysis correlating the GDS values of the samples with their spectral profiles.

Determining the β-sheet protein structures is based on the presence of amyloid peptides which appear in various types of biological tissues of patients with AD. Given the predominant, characteristic, β-sheet structure, it can be determined by infrared spectroscopy using two methods: i) qualitatively and ii) quantitatively, as follows:
i. *Qualitatively.* Proteins and polypeptides with β-structure generate infrared absorption bands in the 1640-1623 cm⁻¹ range, which has been demonstrated by means of spectra on patterns of such biomolecules (Barth, Biochim. Biophys. Acta, 1767, 1073-1101 (2007); Barth and Zscherp, Quart. Rev. Biophysics, 35, 369-430 (2002); Harris and Chapman, Biopolymers, 37, 251-263 (1995)). Accordingly, given that the amyloid peptides associated with AD have a β-polypeptide structure (Rak et al., Biopolymers, 78, 207-217 (2007); Choo et al., Biophys. J., 71, 1672-1679 (1996); Fabian et al., Appl. Spectrosc., 47, 1513-1518 (1993)), the detection of a relative increase of this structure with respect to the controls must be attributed to the presence of these AD biomarkers. Based on the spectroscopic signals measured and amounts of samples collected, amounts of less than 10 µg/ml of these peptides in blood, which is of the same order of magnitude as the sensitivity of the fluorescence and ultraviolet spectroscopy, can be detected by means of this infrared technique (Brown et al., J. Lab. Clin. Med., 137, 5-13 (2001)). This level of detection is due, on one hand, to the method of obtaining the leukocyte cellular fraction, and on the other hand to the fact that amyloid peptides adopt a predominantly β-structure (Hiramatsu and Kitagawa, Biochim. Biophys. Acta, 1753, 100-107 (2005); Zandomeneghi et al., Protein Science, 13, 3314-3321 (2004); Choo et al., Biophys. J., 71, 1672-1679 (1996); Fabian et al., Appl. Spectrosc., 47, 1513-1518 (1993)). Although these peptides become visible with a weak intensity in the shape of a shoulder, the use of second-derivative spectroscopy in the spectral frequency domain unmistakably show the presence of the β-polypeptide structure, as illustrated below.
ii. *Quantitatively.* This method consists of measuring the percentages of β-structure existing in samples of collected blood from patients with AD (and they would correspond to known class II samples of collected blood) and comparing said percentages with those obtained from negative control samples (i.e., known class I samples of collected blood). For this purpose, and for sensitivity reasons, the amide I region (1700-1600 cm⁻¹) expressed in its second-derivative spectrum has been considered in this invention. The percentage of β-structure is determined as a percentage of the area of the bands located in the 1639-1623 cm⁻¹ range with respect to the total area of the spectral profile between about 1680-1623 cm⁻¹. The areas of the individual bands can be calculated by means of the known methods of least squares fitting of profiles or by means of integrating the area enclosed by a spectral profile between two specific frequencies and a baseline cutting said profile into those two frequencies. They are relatively simple methods that are included in current commercial spectroscopic instrument software. It must be pointed out that given the possible interference of atmospheric water vapor in the amide I region of the protein samples, the water vapor spectrum measured under the same instrumental conditions as the samples of leukocytes have to be subtracted. Furthermore, and to eliminate any possible influence of noise on the spectral profiles to be measured, spectra with a good signal-to-noise ratio resulting from averaging at least 32 spectral scans must be obtained.

A second aspect of the invention relates to the method of the present invention to obtain an indication of the presence or absence of the status of the Alzheimer's disease in a sample of collected blood, based on the classification of said sample in the classes or subclasses herein defined. In this sense, if a sample of blood analyzed by this method is classified as class I or subclass I-a, it is indicative of an absence of AD status. Likewise, if said spectroscopic analysis classifies a sample of blood as class II, or any of its subclasses II-a, II-b, II-c, II-d and II-e, said analysis is indicative of a presence of AD status.

Another object of the present invention relates to the method based on multivariate statistical analysis of PLS to obtain an indication of the degree of global cognitive impairment associated with Alzheimer's disease in a sample of collected blood. In this case, the degree of global cognitive impairment directly correlates with the classification in subclasses I-a, II-a, II-b, II-c, II-d and II-e, which allows indicating a GDS value corresponding to the analyzed sample of collected blood.

The method of the present invention can therefore be used for clinical purposes, to assess an individual of interest for diagnosis and/or for determining the degree of progression of Alzheimer's disease, from a sample of blood previously collected from said individual.

A final aspect of the invention relates to a kit for collecting a sample for spectroscopically measuring the global cognitive impairment associated with Alzheimer's disease comprising at least one blood fractionation tube and at least one water-insoluble sample support glass suitable as a window for transmission infrared spectroscopy in the 3100-1300 cm⁻¹ range.

### Detailed Description of the Drawings

Figure 1 shows the 1800-1100 cm⁻¹ region of the transmission infrared spectra of samples of leukocytes from peripheral blood collected from a healthy control (top) and from an AD patient with GDS-7 (bottom).
Figure 2 shows the 1700-1600 cm⁻¹ region of the second-derivative infrared spectra of samples of leukocytes from peripheral blood collected from a healthy control (top) and from an AD patient with GDS 7 (bottom).
Figure 3 shows the percentages of β-protein structure in samples of leukocytes from blood collected from healthy controls (young controls and senile controls) and from AD patients with GDS values that are mild (GDS-3), moderate (GDS-4 and GDS-5) and severe (GDS-6 and GDS-7). The results of each group are expressed by their mean value ± standard error of the mean.
Figure 4 shows the 3100-1100 cm⁻¹ region of the transmission infrared spectra of samples of leukocytes from peripheral blood collected from a healthy control (top) and from an AD patient with GDS-7 (bottom).
Figure 5 shows the comparison between actual GDS values and calculated or predicted GDS values, and the fitting thereof to a linear regression model, Y = A + B*X, where A = 1.7 ± 0.4; B = 0.71 ± 0.1 and R = 0.70.

### Embodiments of the Invention

This invention is illustrated in further detail in the following examples.

### Example 1

### Method for collecting a fraction of leukocytes from peripheral blood

To obtain the fraction of leukocytes from a sample of collected blood, the following steps are carried out:
a) 4 mL of blood are taken, collected from drawing venous blood from healthy controls and from patients with AD in a BD Vacutainer CPT tube with sodium citrate. Such tubes have a fluid gradient and barrier gel to separate erythrocytes and neutrophils to the bottom by centrifugation-filtration, leaving at the top lymphocytes and monocytes (two types of leukocyte blood cells). The tube is centrifuged at 1650 g for 20 minutes at 4°C.
b) The supernatant is collected, the tube is washed with 8 mL of phosphate buffer saline (PBS), and the supernatant and washing liquid combination is centrifuged at 1000 g for 10 minutes at 4°C.
c) The supernatant is discarded, and the sediment is resuspended in 10 mL of PBS which is then centrifuged at 1000 g for 10 minutes at 4°C.
d) The supernatant is discarded, the sediment is resuspended in 1 mL of PBS and centrifuged at 1400 g for 10 minutes at 4°C.
e) The supernatant is discarded, and the sediment is resuspended in 500 µL of PBS. This aqueous suspension is used for preparing samples for infrared spectroscopy.

### Example 2

### Obtaining a leukocyte film on a zinc selenide glass for subsequent infrared analysis

About 100 microliters of the stock suspension containing leukocytes prepared according to the method indicated in Example 1 are collected with a micropipette and deposited on a zinc selenide glass used as a transmission window for infrared spectroscopy.

The purpose of using zinc selenide, which is water-insoluble, is to obtain spectra in the broadest possible spectral range (4000-500 cm⁻¹), unlike what occurs with calcium fluoride glass, which have a narrower window in the infrared (4000-1000 cm⁻¹). Other water-insoluble windows for transmission infrared spectroscopy suitable for analyzing the 3100-1300 cm⁻¹ spectral range can also be valid, such as barium fluoride or zinc sulfide windows as non-limiting examples.

The 100 microliters deposited on the zinc selenide glass are left to evaporate at 4°C. After spreading them out with a spatula to cover a surface of at least 1 cm² and thus prevent, to the extent possible, the leukocyte film from being too thick and therefore causing scattering accompanied by a tilted spectral background affecting the accuracy of the measurements. Furthermore and in relation to it, a suitable thickness for this leukocyte film must generate an amide I band the peak of which is located above 10% transmission.

### Example 3

### Measuring the concentration of β-sheet protein structure by means of second-derivative infrared spectroscopy

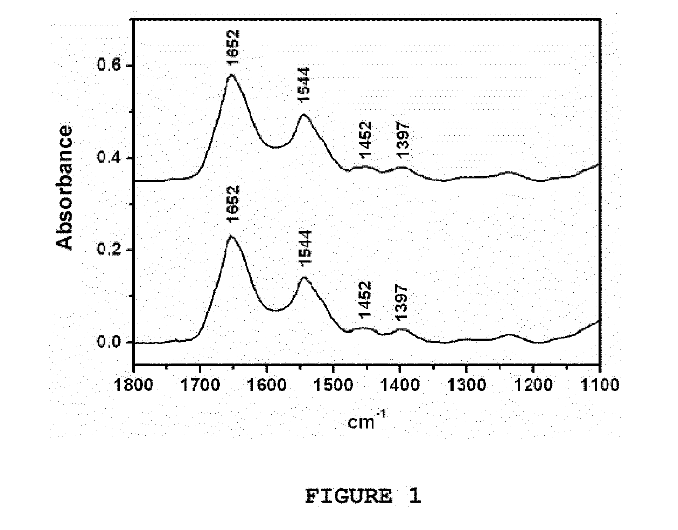

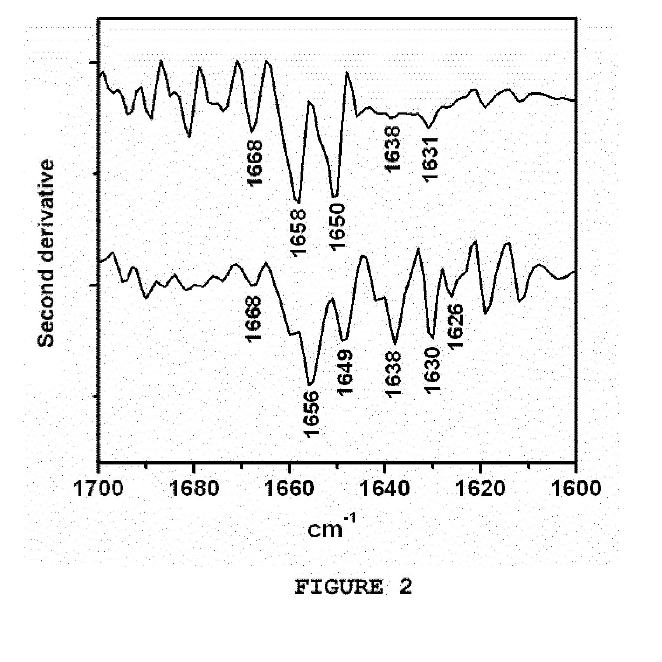

Following the method indicated above for preparing the leukocyte film, a sample of about 100 microliters of aqueous suspension containing leukocytes of each sample is deposited on zinc selenide glass, for which, after subsequent evaporation, the transmission infrared spectrum is measured in a Perkin-Elmer 1725X spectrometer. Each spectrum obtained is the result of averaging 32 scans with a resolution of 2 cm⁻¹ and of subtracting the water vapor spectrum to eliminate possible interferences particularly in the amide I region (1700-1600 cm⁻¹) and amide II region (1560-1520 cm⁻¹). Figure 1 includes spectra in the 1800-1100 cm⁻¹ region from an analyzed sample of blood collected from a healthy control and from an AD patient with GDS-7. These spectra are very similar, and therefore it is necessary to use their spectra expressed in second derivatives which are included in Figure 2. It must be pointed out that an important difference between these last two spectra lies in the region where bands caused by β-structures of polypeptides and proteins (1640-1620 cm⁻¹) appear. The increase in intensity of these bands in samples from patients with AD can be attributed to the presence of amyloid peptides. For the purpose of determining the relative concentrations of this structure in samples of blood collected from AD patients and comparing them with samples from healthy individuals, such concentrations were measured in samples of leukocytes collected from a control groups and patients with various degrees GDS of this disease. This group of samples of collected blood is indicated in Table 1, where the values of the GDS criterion for each group of patients and controls studied and the number of subjects for each group are included. The group called young control includes healthy individuals (GDS-1) whose ages are comprised between 20 and 30 years old, unlike the group called senile control and the groups with Alzheimer's disease, all corresponding to ages greater than 65 years old.

**Table 1**

| GROUPS | DEGREE | Total Patients |
|---|---|---|
| Young controls | GDS 1 | 6 |
| Senile controls | GDS 1 | 30 |
| mild AD | GDS 3 | 15 |
| moderate AD | GDS 4 | 15 |
| | GDS 5 | 14 |
| severe AD | GDS 6 | 13 |
| | GDS 7 | 13 |

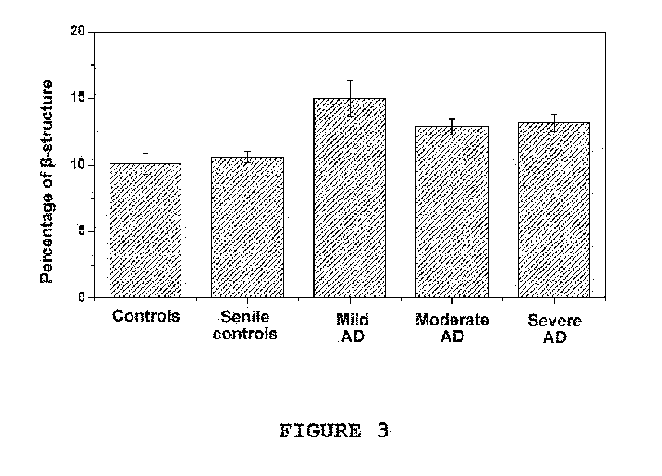

The percentages of β-structure measured as described in the detailed description are included in Figure 3 after regrouping them into five AD groups or levels (according to the GDS criterion), namely, young controls, senile controls, mild AD, moderate AD, and advanced or severe AD.

It can be inferred from these results that, on one hand, there is no clear difference between β-protein structure levels of young controls and senile controls, and on the other hand, the mean concentrations of each of the groups with AD are greater than that of any control group. Furthermore, the highest percentages of β-structure are generated in the mild AD level, and then they decrease in the course of the disease. Accordingly, with respect to the controls, the samples of mild AD are expected to be more readily differentiated than the samples of moderate and severe AD. The corresponding ROC (Receiver Operating Characteristic) curves, which have been obtained for the samples of mild AD in Table 1 and express the sensitivity as a function of specificity, allow attributing a specificity of about 82% for a sensitivity of about 81% to said spectroscopic method for determining β-sheet protein structures.

### Example 4

### Multivariate statistical analysis of partial least squares (PLS) of infrared spectra of leukocytes

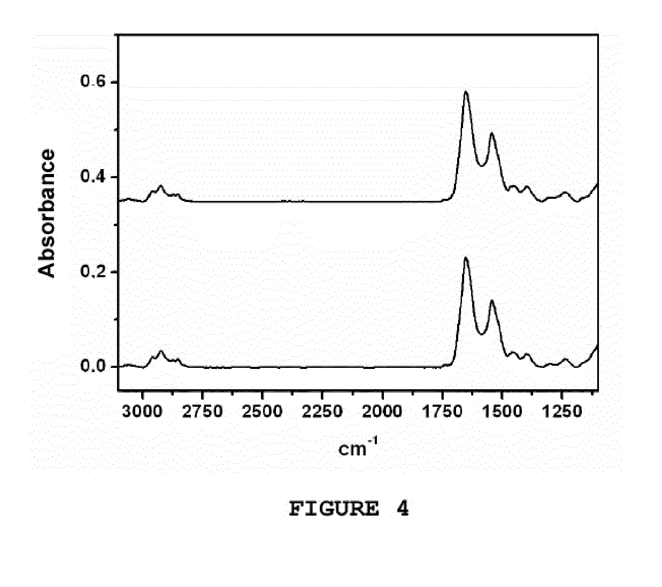

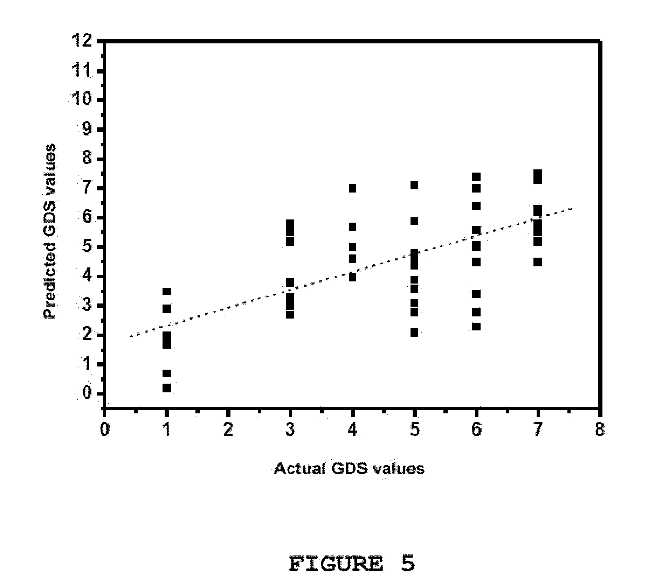

A multivariate statistical analysis of PLS has been carried out based on the group of samples indicated in Table 1. The C-H stretching spectral regions (3020-2830 cm⁻¹) and the 1720-1350 cm⁻¹ region comprising intense amide I and amide II bands, and mean intensity bands towards 1450 and 1400 cm⁻¹ were considered for this purpose. The spectra subjected to this statistical processing were the result of correcting the baseline, as shown in Figure 4, normalizing them with respect to the height of the amide I band and all of them referring to the same absorbance for the 1800 cm⁻¹ frequency. The AD GDS criterion was considered as a dependent variable, and the absorbances in each of the frequencies of said regions were considered as independent variables. To predict the GDS values a cross-assessment was performed considering all the samples except for the analytical model, and the excluded sample was used to predict its GDS value. Such prediction with a cross-assessment was performed considering twelve components at most for each of the samples making up the group of Table 1. The results are shown in Figure 5, which shows the predicted GDS values versus the actual values. It can be seen in this drawing that predicted GDS values greater than 1 are obtained for some control samples (GDS-1), and, inversely, for some samples associated with AD the method of PLS predicts GDS values around 1 or 2, which are values that can be confused with the values of the controls. These results show method sensitivity (80%) and specificity (65%). In this case, sensitivity refers to the probability that a sample from a type II subclass (II-a, II-b, II-c, II-d or II-e) subjected to analysis by this method will give a positive result for subclass II and in percentage terms it is calculated as the number of hits of type II samples divided by the total number of type II samples analyzed and by multiplying the resulting quotient by 100. In terms of specificity, it refers to the probability that a subclass I-a sample subjected to analysis by this method will give a positive result for I-a type subclass, and in percentage terms it is calculated as the number of hits of subclass I-a samples divided by the total number of subclass I-a samples analyzed and by multiplying the resulting quotient by 100.

## Claims

1. An infrared spectroscopic method for determining the global cognitive impairment associated with Alzheimer's disease, **characterized in that** it comprises the following steps:
a. recording a transmission infrared spectrum of a first sample of collected blood;
b. obtaining at least a first numerical parameter from at least one signal of the spectrum of the region comprised between 3100 and 1300 cm⁻¹, including both limits;
c. comparing said at least first numerical parameter of step 1b with at least a second reference numerical parameter;
d. determining based on the comparison in the preceding step if the sample of blood of step 1a corresponds to a class defined by:
I. no cognitive impairment associated with Alzheimer's disease,
II. cognitive impairment associated with Alzheimer's disease.

2. The method according to claim 1, **characterized in that** the first sample of collected blood is a sample collected from human peripheral blood.

3. The method according to any one of claims 1 or 2, **characterized in that** the first sample of blood is a sample of a fraction of collected blood selected from a cellular fraction and an acellular fraction.

4. The method according to claim 3, **characterized in that** the fraction of collected blood is a fraction of leukocytes in human peripheral blood.

5. The method according to any one of claims 3 or 4, **characterized in that** the sample of a fraction of collected blood is prepared following a method which comprises:
a. preparing an aqueous suspension of a fraction of collected blood by centrifugation-filtration,
b. depositing and spreading a volume of the aforementioned aqueous suspension comprised between 5 and 125 µL, including both limits, over a zinc selenide glass for infrared spectroscopy,
c. completely evaporating the deposited suspension at temperatures comprised between 2 and 20°C, including both limits.

6. The method according to any one of claims 1 to 5, **characterized in that** said first numerical parameter is a percentage of β-peptide structure content of the sample of collected blood.

7. The method according to claim 6, **characterized in that** the percentage of β-peptide structure content is obtained by a method which comprises:
a. expressing an infrared spectrum region comprised between 1700 and 1600 cm⁻¹, including both limits, in the second derivative in the frequency domain;
b. identifying each of the bands associated with the β-peptide structure in the infrared spectrum region comprised between 1640 and 1620 cm⁻¹, including both limits;
c. calculating the percentage of β-peptide structure by multiplying by 100 the sum of the areas of each of the bands associated with a β-peptide type structure in the infrared spectrum region comprised between 1639 and 1623 cm⁻¹, including both limits, and dividing by the total area of said spectrum in the region comprised between 1680 and 1623 cm⁻¹, including both limits.

8. The method according to any one of claims 6 or 7, **characterized in that** the percentage of β-peptide structure content of the infrared spectrum classifies the first sample of collected blood as follows:
a. sample of blood corresponding to class I if the percentage of β-peptide structure content is equal to or less than 12%;
b. sample of blood corresponding to class II if the percentage of the β-structure content is greater than 12%.

9. The method according to any one of claims 1 to 5, **characterized in that** the at least second reference numerical parameter is obtained under the same conditions as the first parameter of step 1b, from a second infrared spectrum recorded in at least a second reference sample of blood collected from each of the classes defined in I and II.

10. The method according to claim 9, **characterized in that**:
a. class I of said second reference sample of collected blood comprises at least the subclass defined by:
I-a. no cognitive impairment associated with Alzheimer's disease with a value of GDS-1 on Reisberg's global deterioration scale,
b. class II of said second reference sample of blood comprises at least the subclasses defined by:
II-a. mild cognitive impairment associated with Alzheimer's disease with a value of GDS-3 on Reisberg's global deterioration scale,
II-b. moderate cognitive impairment associated with Alzheimer's disease with a value of GDS-4 on Reisberg's global deterioration scale,
II-c. moderately severe cognitive impairment associated with Alzheimer's disease with a value of GDS-5 on Reisberg's global deterioration scale,
II-d. severe cognitive impairment associated with Alzheimer's disease with a value of GDS-6 on Reisberg's global deterioration scale,
II-e. very severe cognitive impairment associated with Alzheimer's disease with a value of GDS-7 on Reisberg's global deterioration scale.

11. The method according to claim 10, **characterized in that** said first numerical parameter is the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of the first sample of blood, and said second reference numerical parameter of each of the classes defined in I-a, II-a, II-b, II-c, II-d and II-e is the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of each reference sample of blood.

12. The method according to claim 11, **characterized in that** the absorbance values of each of the frequencies of the regions comprised between 1500-1300 cm⁻¹, 1600-1500 cm⁻¹, 1700-1600 cm⁻¹, and 3100-2800 cm⁻¹ are determined from the spectrum previously normalized with respect to the absorbance of the amide I band in the 1700-1600 cm⁻¹ region, and referring to one and the same absorbance value in the 1800 cm⁻¹ frequency.

13. The method according to claim 12, **characterized in that** the comparison of step 1d comprises a partial least squares multivariate analysis using:
- the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of the first sample of blood and the absorbance values of each of the frequencies of the regions comprised between 1500-1300 cm⁻¹, 1600-1500 cm⁻¹, 1700-1600 cm⁻¹, and 3100-2800 cm⁻¹ of the spectrum of each reference sample of blood as independent variables;
- and the numerical GDS value on Reisberg's global deterioration scale corresponding to the subclass defined in I-a, II-a, II-b, II-c, II-d and II-e of said second reference sample of blood as a dependent variable.

14. The method of any one of claims 1 to 13 to obtain an indication of the presence or absence of the status of the Alzheimer's disease in a sample of collected blood.

15. The method of any one of claims 10 to 13 to obtain an indication of the degree of global cognitive impairment associated with Alzheimer's disease in a sample of collected blood.

16. Kit for collecting a sample for spectroscopically measuring the global cognitive impairment associated with Alzheimer's disease, comprising at least one blood fractionation tube and at least one water-insoluble sample glass support suitable as a window for transmission infrared spectroscopy in the 3100-1300 cm⁻¹ range.
